# EUROPÄISCHE PATENTSCHRIFT

(11) **EP 0 434 806 B1**
(45) Veröffentlichungstag und Bekanntmachung des Hinweises auf die Patenterteilung: **17.04.1996**
(21) Anmeldenummer: 90910591.8
(22) Anmeldetag: 16.07.1990
(51) Int. Cl.: A61N 1/365

(54) **HERZSCHRITTMACHER MIT AKTIVITÄTSSENSOR**
PACEMAKER WITH ACTIVITY SENSOR
STIMULATEUR CARDIAQUE AVEC CAPTEUR D'ACTIVITE

(30) Priorität: 15.07.1989 DE 3923801
(43) Veröffentlichungstag der Anmeldung: 03.07.1991
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-8520 Erlangen (DE)
(74) Vertreter: Christiansen, Henning, Dipl.-Ing.
(86) Internationale Anmeldenummer: DE9000543
(87) Internationale Veröffentlichungsnummer: WO9101157

(56) Entgegenhaltungen:
- EP-A- 0 178 528
- EP-A- 0 203 027
- EP-A- 0 331 309

## Beschreibung

Die Erfindung betrifft einen physiologisch arbeitenden, ratengesteuerten Herzschrittmacher.

Ein derartiger Herzschrittmacher ist beispielsweise aus der US-A-4 140 132 oder der EP-A-0 080 348 bekannt. Der dort beschriebene Herzschrittmacher enthält einen Aktivitätssensor, der die Bewegungsintensität des Patienten detektiert. Eine Auswerteschaltung mißt das Ausgangssignal beispielsweise eines Biegeschwingers und wertet die spektralen Spitzen des durch Tätigkeiten des Patienten erzeugten Ausgangssignals oberhalb einer vorbestimmten programmierbar vorgebbaren Schwelle aus. In Abhängigkeit von diesen detektierten, mit der jeweiligen Tätigkeit des Patienten veränderlichen, Aktivitätspegeln werden die Grundrate des Schrittmachers und damit - im Falle eines Demand-Schrittmachers - die Escapesequenzen des Schrittmachers zwischen vorgegebenen Minimal- und Maximalwerten variiert.

Der Erfindung liegt die Aufgabe zugrunde, einen implantierbaren Herzschrittmacher anzugeben, der aus dem Ausgangssignal eines Aktivitätssensors, speziell Biegeschwingers, einen Grundratenwert (Escapesequenz im Falle der Demand-Stimulation) ableitet, der eng an den tatsächlichen physiologischen Bedarf des Patienten gekoppelt ist.

Diese Aufgabe wird durch die im Anspruch 1 angegebenen Merkmale gelöst. Vorteilhafte Weiterbildungen der Erfindung sind in den Unteransprüchen wiedergegeben.

Die Erfindung beruht auf der Erkenntnis, daß eine Auswertung des Verhältnisses der Zeitdauer des Verweilens des bandbegrenzten Ausgangssignals des Aktivitätssensors oberhalb eines programmierten Schwellwertes zur Zeitdauer des Verweilens unterhalb des Schwellwertes bestimmt wird. Das bedeutet, daß das aus den für die jeweilige Tätigkeit des Patienten repräsentativen Aktivitätssignalen abgeleitete Ausgangssignal der Auswerteschaltung abhängig von der Amplitude der Impulse ist. Daher ist die aus dem Ausgangssignal abgeleitete Escapesequenz stets besonders eng an den tatsächlichen aktuellen physiologischen Bedarf des Patienten gekoppelt.

Bei der bevorzugten Ausführung des erfindungsgemäßen Herzschrittmachers werden von einer Auswerteschaltung entsprechend der ermittelten Aktivität des Patienten Steuersignale für Stimulationsimpulse erzeugt, die aus einem Verhältnis der gewichteten Zeitmittel der Zeitdauer oberhalb eines Schwellwertes zu der Zeitdauer unterhalb des Schwellwertes eines bandbegrenzten momentanen Sensorausgangssignals detektiert werden.

Im Gegensatz zu der aus der EP-A-0 080 348 bekannten Bandbegrenzung, bei der nur Frequenzanteile zwischen 10 bis 100 Hz passieren können, wird bei einer bevorzugten Ausführungsform des erfindungsgemäßen Herzschrittmachers ein Bandpaßfilter verwendet, der für die durch die Frequenz der Bewegung des Patienten selbst erzeugten Ausgangssignale des Sensors durchlässig ist, insbesondere für Frequenzen im Bereich von 2 bis 8 Hz.

Die Gewichtungsfaktoren, mit denen die gefilterten Ausgangsimpulse des Sensors bewertet werden, können bei dem erfindungsgemäßen Herzschrittmacher für die momentanen Signalpegel oberhalb und unterhalb der vorgegebenen Schwelle verschieden sein. Diese sind so gewählt, daß die in einer nachfolgenden Stufe gemittelten Impulse bei Überschreitung der Schwelle ein Steuersignal bilden, welches mit relativ großer Steigung eine Zunahme der Stimulationsgrundrate bewirkt, während bei Unterschreitung im Mittel die Abnahme der Stimulationsgrundrate mit geringerer Steigung erfolgt - wobei die betreffenden Gewichtungsfaktoren in Anpassung an die natürlichen physiologischen Gegebenheiten gewählt sind, entsprechend dem Anstieg der Herzfrequenz bei einer körperlichen Belastung, wie sie zu den aufgenommenen Aktivitätssignalen gehört, bzw. entsprechend dem Abfall der Herzrate beim Wegfall dieser Belastung. Die Gewichtungsfaktoren sind bei einer anderen vorteilhaften Weiterbildung der Erfindung durch Programmierung veränderbar und korrespondieren insoweit also mit den programmierten Zahlenwerten der Anstiegs- und Abfallzeiten (jeweils bezogen auf vorbestimmte Herzratendifferenzen).

Wenn die Signalform während der Zeit oberhalb der Schwelle mit der Ausgangsamplitude des Sensors ansteigt, ist die Stimulationsratenregelung abhängig von der Mittelwertamplitude der Schwingungen innerhalb der Zeit oberhalb der Schwelle.

Ein Ausführungsbeispiel der Erfindung wird nachstehend anhand der Zeichnung erläutert. Es zeigen:
Fig. 1 einen bei einer Ausführungsform des erfindungsgemäßen Herzschrittmachers als Sensor verwendeten Biegeschwinger,
Fig. 2a das programmierbar veränderbare Ratenverhalten des Schrittmachers bei zunehmender Aktivität,
Fig. 2b das programmierbar veränderbare Ratenverhalten des Schrittmachers bei abnehmender Aktivität sowie
Fig. 3 ein Blockschaltbild der Signalverarbeitungsschaltung.

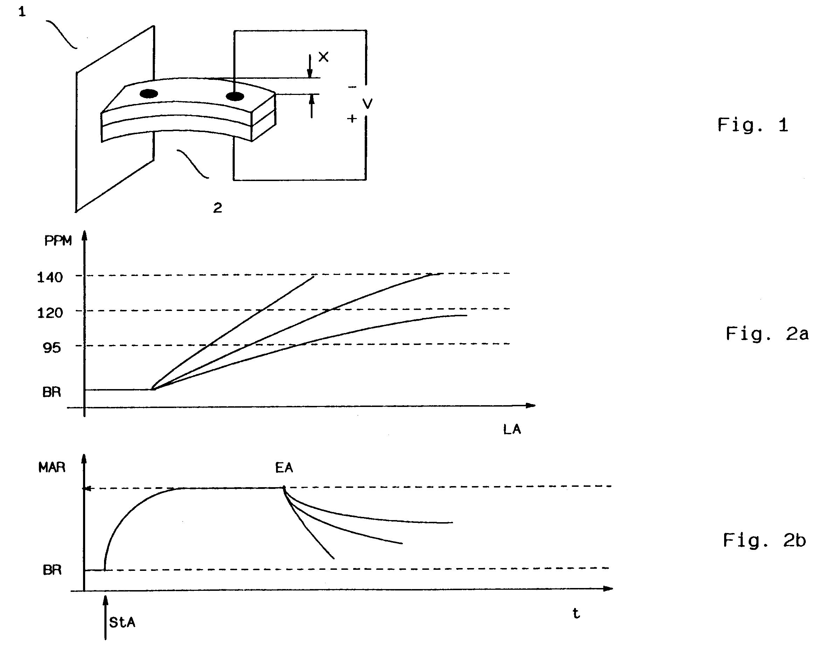

Fig. 1 zeigt einen Biegeschwinger 2, der innerhalb des Herzschrittmachergehäuses 1 angebracht ist. Ein solcher Biegeschwinger 2 kann beipielsweise ein piezokeramischer Streifen sein, dessen Auslenkungen x, hervorgerufen durch Schalldruckwellen der Tätigkeiten des Patienten, mittels eines Wandlers in entsprechende Spannungsamplituden umgewandelt werden. Der Biegeschwinger 2 bildet aufgrund seiner Frequenzcharakteristik selbst die Hochpaßflanke zur Bandbegrenzung des Eingangssignals. Die Tiefpaßflanke wird durch ein nachgeschaltetes, mittels eines Differenzverstärkers realisiertes Eingangsfilter gebildet. Der so erzeugte Bandpaß hat einen Durchlaßbereich von 2 bis 8 Hz bei einer Güte Q = 0,707.

Fig. 2 zeigt das Ratenregelverhalten in Abhängigkeit von der Aktivität des Patienten, wobei es sich im strengen Sinne wegen der fehlenden Rückkopplung um eine Steuerung handelt. Es werden zwei verschiedene Regelparameterkurven verwendet mit je drei programmierbaren Anfangswerten. Fig. 2a zeigt die Regelparameterkurven der Anstiegszeit, Fig. 2b die Regelparameterkurven der Abfallzeit der Aktivität des Patienten. Je nach Erfordernis für den Patienten kann der Grundherzschlag BR 62, 72 oder 82 Schläge pro Minute PPM (Fig. 2a) sowie die maximal programmierbare Aktivitätsrate MAR 100, 125 oder 150 Schläge pro Minute betragen. Die erzeugte Impulsrate ist linear abhängig von der Pulsratenregelspannung, die eine Funktion der gewählten Regelparameterkurven ist. Über der Abszisse in Fig. 2a ist das Ansteigen des Aktivitätsniveaus, über der Abszisse in Fig. 2b ist die Zeit aufgetragen. Wenn die Spannung des Sensors größer ist als die Schwellenspannung, erhöht sich die Pulsratenregelspannung auf einen Wert, der aus der festgelegten Einregelungszeit ermittelt wird, wobei diese programmierbare Anstiegszeit einen Wert von 2, 4 oder 8 Sekunden haben kann. Wenn die Spannung des Sensors kleiner ist als die Schwellenspannung, sinkt die Stimulationsratenregelspannung um einen Wert, der anhand der vorgegebenen Abfallzeit ermittelt wird, wobei diese programmierbare Zeit einen Wert von 30, 50 oder 100 Sekunden haben kann und auf die Rückkehr zur Grundrate bei Wegfall der körperlichen Last bezogen ist.

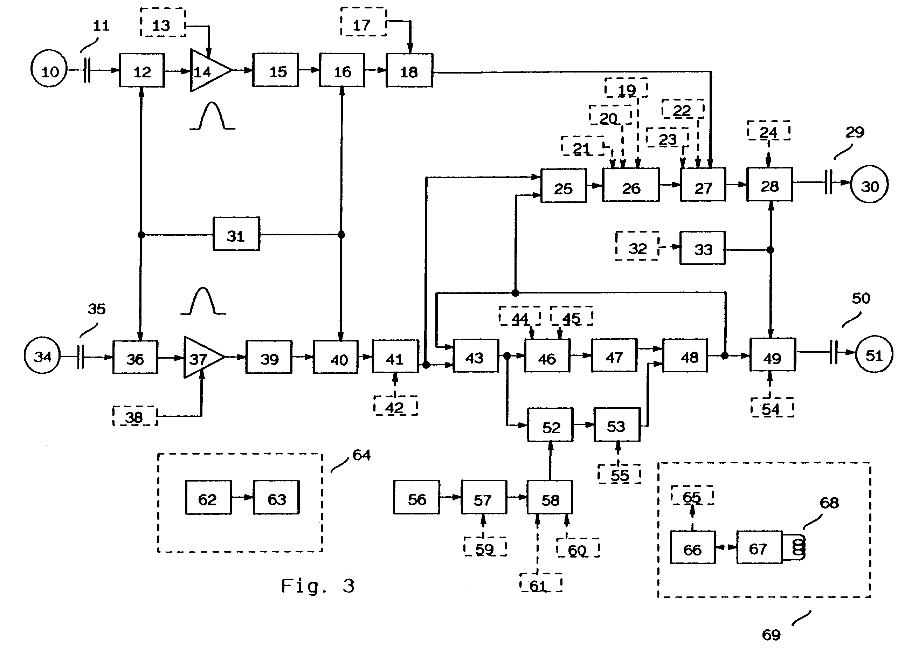

Fig. 3 zeigt ein Blockschaltbild der Signalverarbeitungsschaltung, wobei der Zweikammer-Schrittmacher im DDD-Modus arbeitet, d. h. daß die physiologische Stimulation den Vorhof (Atrium) in einen zyklischen Steuerungsablauf mit einbezieht. Die beiden Eingangs-Kanäle für Ventrikel und Atrium sind sehr ähnlich aufgebaut. Das Ventrikeldetektionssignal 10 wird über einen Kondensator 11 zur Gleichspannungsunterdrückung einer V-Austastschaltung 12 zugeführt. Diese Austastschaltung 12 blendet das V-Eingangssignal während eines Ausgangsimpulses aus und entlädt den Kondensator 11 nach dem Ausgangsimpuls. Das Ausgangssignal der Austastschaltung 12 wird einem V-Signal-Verstärker 14 zugeführt, dessen Verstärkungsfaktor variabel ist und eine Bandpaßcharakteristik aufweist mit einer Mittenfrequenz von 50 Hz. Die V-Empfindlichkeit 13 ist von außen programmierbar. Der Ausgang des V-Signal-Verstärkers 14 wird einem V-Detektor 15 zugeführt. Dieser V-Detektor 15 kann ein bipolarer Spitzenwertgleichrichter mit einer fest eingestellten Detektionsschwelle sein. Die Verstärkung des V-Signal-Verstärkers 14 wird so eingestellt, daß das zugehörige Eingangssignal detektierbar ist.

Das digitale Ausgangssignal des V-Detektors 15 wird einem V-Signal-Störspannungsbegrenzer 16 zugeführt. Eingangssignale mit einer Periode kleiner als 125 ms werden ausgeblendet. Der V-Signal-Störspannungsbegrenzer 16 enthält zusätzlich eine Austastung des digitalen Signals während eines Ausgangsimpulses. Dazu ist der V-Signal-Störspannungsbegrenzer 16 mit einer Eingangstaktschaltung 31 verbunden. Das Ausgangssignal des V-Signal-Störspannungsbegrenzers 16 führt auf eine V-Refraktärzeitschaltung 18 mit programmierbarer Refraktärzeit 17. Diese V-Refraktärzeitschaltung 18 ist nicht rücksetzbar. Nach Detektion eines Eingangssignals für die programmierte Refraktärzeit wird somit von der Schaltung kein weiteres Eingangssignal mehr detektiert. Das Ausgangssignal der Refraktärzeitschaltung 18 wird einer Taktantwortschaltung 27 zugeführt.

Das Atriumdetektionssignal 34 wird über einen Kondensator 35 zur Gleichspannungsunterdrückung einer A-Austastschaltung 36 zugeführt. Das Ausgangssignal der A-Austastschaltung 36 wird einem A-Signal-Verstärker 37 zugeführt, dessen Verstärkungsfaktor 38 programmierbar ist und eine Bandpaßcharakteristik aufweist mit einer Mittenfrequenz von 100 Hz. Der Ausgang des A-Signal-Verstärkers 37 wird einem A-Detektor 39 zugeführt. Das digitale Ausgangssignal des A-Detektors 39 ist verbunden mit einem A-Signal-Störspannungsbegrenzer 40. Der A-Signal-Störspannungsbegrenzer 40 ist mit der Eingangstaktschaltung 31 verbunden. Das Ausgangssignal des A-Signal-Störspannungsbegrenzers 40 führt auf eine A-Refraktärzeitschaltung 41 mit programmierbarer Refraktärzeit 42. Zur Ausblendung der Eingangssignale sind die Austastschaltungen 12, 36 mit der Eingangstaktschaltung 31 verbunden. Der Aufbau des A-Kanals entspricht dem des V-Kanals.

Als Ausgangssignal beider Kanäle nach den Refraktärzeitschaltungen 18, 41 wird ein digitaler Impuls erzeugt, der aus einem detektierten Ereignis entweder im Atrium oder Ventrikel des Herzens abgeleitet ist. Beide Eingangskanäle haben die Funktion, die Eingangssignale zu überprüfen, die von den Atrium- bzw. Ventrikeldetektoren 10, 34 der Schaltung zugeführt werden, um sicherzustellen, daß alle gewünschten Herzereignisse detektiert werden, während Störsignale ausgeblendet werden.

Das Ausgangssignal der A-Refraktärzeitschaltung 41 wird einer ODER-Schaltung 43 sowie einer ODER-Schaltung 25 zugeführt. Das Ausgangssignal der ODER-Schaltung 43 wird sowohl einer Taktzentrale 46 als auch einer Aktivitätstaktschaltung 52 mit spannungsabhängigem Oszillator zugeführt. Die Taktzentrale 46 ist ein rücksetzbarer Aufwärtszähler, dessen Taktrate 44 und Hysterese 45 programmierbar sind. Dieser Aufwärtszähler wird getaktet durch einen Quarzoszillator. Dieser Aufwärtszähler der Taktzentrale 46 liefert ein Ausgangssignal, wenn der Wert des Aufwärtszählers der Taktzentrale 46 den decodierten Wert der programmierten Taktrate 44 erreicht. Dieses Ausgangssignal, welches über eine Schutzschaltung 47 zur Vermeidung zu hoher Taktraten geführt wird, dient dazu, den Aufwärtszähler der Taktzentrale 46 für eine erneute Zählperiode zurückzusetzen. Dazu wird das Ausgangssignal über eine ODER-Schaltung 48 auf einen weiteren Eingang der ODER-Schaltung 43 geführt. Die Schutzschaltung 47 besteht aus einer nicht rücksetzbaren monostabilen Kippschaltung, welche die maximale Taktrate der Taktzentrale 46 auf einen festen Wert begrenzt, falls diese fehlerhaft arbeitet. Somit ist diese Taktzentrale 46 freilaufend und erzeugt ein Ausgangssignal auf den Wert der programmierten Taktraten. Zusätzlich zum Rücksetzen durch seinen eigenen Impuls kann der Aufwärtszähler der Taktzentrale 46 durch das Ausgangssignal des Atrium-Eingangskanals zurückgesetzt werden. Um zu verhindern, daß der Aufwärtszähler der Taktzentrale 46 über längere Zeit durch externes Zurücksetzen bei einer höheren als programmierten Taktrate kein Ausgangssignal liefert, kann die Hysterese 45 programmiert werden. Dadurch wird die programmierte Taktrate 44 ein wenig modifiziert, abhängig davon, ob die letzte Zurücksetzung vom Aufwärtszähler der Taktzentrale 46 oder von dem Atriumeingangskanal veranlaßt wurde.

Das Ausgangssignal des spannungsabhängigen Oszillators 52, dem eine Schutzschaltung 53 mit programmierbaren Maximaltaktrate 55 nachgeschaltet ist, die die Taktrate auf eine aus der maximalen Aktivität abgeleiteten Taktrate begrenzt, ist ebenfalls mit der ODER-Schaltung 48 verbunden. Somit können sowohl die Taktzentrale 46 als auch der spannungsabhängige Oszillator der Aktivitätstaktschaltung 52 durch dieselben Signale zurückgesetzt werden.

Die Taktrate des spannungsabhängigen Oszillators der Aktivitätstaktschaltung 52 ist abhängig von einem Signal, welches aus einem Aktivitätssensor 56 mit nachgeschaltetem Schwellwertdetektor 57 und Signalintegrator 58 abgeleitet wird. Der Schwellwert 59 des Schwellwertdetektors 57 ist durch externe Programmierung veränderlich. Bei dem Signalintegrator 58 können Anstiegszeit 61 und Abfallzeit 60 programmiert werden. Der spannungsabhängige Oszillator 52 kann dazu verwendet werden, die Taktzentrale 46 zu übersteuern und den Ausgangstakt ansteigen zu lassen, bis hin zum maximalen programmierten Aktivitätstakt.

Wenn der externe Rücksetzimpuls der Zähler 46, 52 von dem Atrium-Kanal erzeugt wird, ist der Schrittmacher P-Wellen- oder atriumsynchronisiert. Diese Synchronisierungsart besteht so lange, wie der Atriumkanal einen solchen Ausgangstakt liefert, der höher als der programmierte Takt oder der aus der Aktivität abgeleitete Takt ist. In diesem Fall liefern die Zähler 46, 52 kein Ausgangssignal und sind somit gesperrt.

Die Ausgänge der Zähler 46, 52 sind weiter über die ODER-Schaltung 48 mit einer A-Ausgangsschaltung 49 mit programmierbarer A-Pulsbreite 54 verbunden. Das so erzeugte Atriumstimulationssignal 51 wird über einen Kondensator 50 zur Atriumstimulation dem Herzen zugeführt. Der Ausgang der ODER-Schaltung 25 ist mit einer programmierbaren A-V-Verzögerungsschaltung 26 verbunden. Das Ausgangssignal der ODER-Schaltung 25, welches die A-V-Verzögerungsschaltung 26 startet, repräsentiert entweder ein detektiertes Atrium-Ereignis oder das Ausgangssignal eines der Zähler 46, 52, die ermittelten, daß ein Atrium-Ereignis eingeleitet werden sollte. Die programmierbare A-V-Verzögerungszeit 21 repräsentiert die Zeit zwischen einem synchronisierten Atrium- und Ventrikularereignis. Der A-V-Rückfall 19 (fallback) ist die programmierte Differenz in der A-V-Verzögerung, abhängig davon, ob die A-V-Verzögerung eingeleitet wurde entweder durch ein gemessenes Atrium-Ereignis oder durch eine Zählerunterbrechung, welche ein getaktetes Atrium-Ereignis einleiten will. Ein programmierbares V_{Com}/U_{Com} Programmbit 20 entscheidet, ob der Schrittmacher umgeschaltet wird auf ein Ventrikular-Ausgangssignal am Schluß der A-V-Verzögerung, wenn ein gemessenes Ventrikularereignis während dieser Zeit stattfand.

Der Ausgang der A-V-Verzögerung 26 ist mit der Taktantwortschaltung 27 verbunden. Dieses Ausgangssignal wird entsprechend der programmierten A-V-Verzögerungszeit erzeugt. Zu diesem Zeitpunkt wird das Ausgangssignal des Ventrikel-Eingangskanals dazu verwendet, ein Ventrikel-Impulssignal zu unterdrücken, wenn ein Ventrikelereignis detektiert wurde. Wenn kein Ventrikelereignis detektiert wurde, wird durch die Stimulationsantwort die obere Trackingrate 23, welche programmierbar ist, gesetzt. Diese obere Trackingrate ist die höchste Rate, mittels der aufeinanderfolgende Ventrikel-Ausgangsimpulse durchgelassen werden können. Die Antwort auf dieser nach oben begrenzten Taktrate ist ebenfalls programmierbar. Dazu kann entweder ein 2:1-Teiler 22 oder eine flache Wenckebach-Ausgangstaktschaltung mit der höchsten programmierten Rate verwendet werden. Das Ausgangssignal der Taktantwortschaltung 27 wird als Impuls einer V-Ausgangsschaltung 28 mit programmierbarer V-Pulsbreite 24 zugeführt. Das so erzeugte Ventrikelstimulationssignal 30 wird über einen Kondensator 29 zur Ventrikelstimulation dem Herzen zugeführt.

Die A-Ausgangsschaltung 49 und die V-Ausgangsschaltung 28 sind ähnlich aufgebaut. Die Eingangsimpulse dieser Ausgangsschaltungen 28, 49 werden dazu verwendet, die negative Flanke des Ausgangsimpulses mit programmierter Pulsbreite zu triggern. Die Amplituden des V- bzw. A-Ausgangsimpulses sind abhängig von der Spannung eines Ladekondensators 32 einer Ladungspumpen-Schaltung 33 (Charge Pump). Diese Spannung ist programmierbar und gleich für beide Ausgangsschaltungen 28, 49.

Der Aktivitätssensor 56, der Schwellwertdetektor 57 und der Signalintegrator 58 ergeben den Aktivitätsschaltkreis, der ein Kontrollsignal für den spannungsabhängigen Oszillator 52 liefert. Der Aktivitätssensor 56 ist ein piezoelektrisches Element, welches vorzugsweise an der Innenseite des Schrittmachergehäuses angebracht ist. Als Ausgangssignal des Sensors 56 wird eine Spannung erzeugt, die proportional zur Auslenkung ist, die durch Körperbewegungen und Stöße hervorgerufen wird. Das Ausgangssignal wird dem Schwellwertdetektor 57 zugeführt, der eine programmierbare Schwelle besitzt. Die integrierte Spannung wird zur Regelung des spannungsabhängigen Oszillators der Aktivitätstaktschaltung 52 verwendet. Der Wert der Stimulationsrate, auf die die Spannung ansteigen kann, ist ein Ergebnis des ansteigenden Eingangssignals und wird Anstieg (attack) genannt und ist programmierbar.

Der Wert der Stimulationsrate, auf die die Spannung abfallen kann, wird Abfall (decay) genannt und ist ebenfalls programmierbar. Die Steuergröße 59 dient zur programmierbaren Veränderung der wirksamen Schwelle der Schwellwertschaltung. Die Steuergrößen 60 und 61 repräsentieren die programmierbaren Gewichtungswerte für die Wirkung der Zeiten der Schwellenüber- bzw. -unterschreitung im Hinblick auf den Integrator 58. Wird der Integrator beispielsweise durch einen Kondensator gebildet, beeinflussen die Steuergrößen den Lade- bzw. Entladestrom. Wird der Kondensator im Falle der Schwellenüberschreitung geladen und bei Schwellenunterschreitung entladen, so stellt sich jeweils eine mittlere Spannung ein, welche die Eingangsspannung für den spannungsabhängigen Oszillator 52 bildet. Bei im Verhältnis vermehrter Schwellenüberschreitung (große Aktivität) steigt die Spannung an und fällt bei verminderter Aktivität wieder ab. Die programmierten Werte von Lade- und Entladegröße bestimmen dabei die Anstiegs- bzw. Abfallkonstante (vgl. Figuren 2a und 2b).

Zwischen Schwellwertdetektor 57 und Integrator 58 ist bei einer nicht dargestellten bevorzugten Weiterbildung ein Verstärker vorgesehen, welcher die die Schwelle überschreitenden Impulse - gegebenenfalls bis zum Begrenzungseinsatz - verstärkt. Auf diese Weise läßt sich der Einfluß der verschiedenen Impulsamplitudenklassen auf die Grundrate des Herzschrittmachers wegen der damit verbundenen nichtlinearen Verzerrung noch weitergehend beeinflussen. Durch geeignete Wahl der Wertes (Kapazität) des Integrators bleibt das Wechselspiel zwischen Aufladung bei Schwellenüberschreitung und Entladung bei Schwellenunterschreitung kurzfristig ohne merkbaren Einfluß auf die Stimulationsrate. Die Veränderung wirkt sich jeweils erst über eine größere Anzahl von Über- und Unterschreitungszyklen aus.

Eine Überwachungsschaltung 62 prüft die Kapazität der Batterie und erzeugt ein EOL-Signal (end of life), wenn die Kapazität unter einen bestimmten Wert fällt. Dieses Signal wird dazu verwendet, den Schrittmacher in eine EOL-Arbeitsweise zu setzen. Das bedeutet, daß der Aktivitätsschaltkreis außer Funktion gesetzt wird und die Teilerschaltung des Quarzoszillators 63 modifiziert wird, so daß alle Taktschaltungen ca. 11% langsamer laufen.

Ein Programmspeicher 66 speichert die Programmbits 65, welche die verschiedenen Parameter des Schrittmachers überwachen. Eine Übertragungsschaltung 67 mit angeschlossenem Übertrager 68 ist als bidirektionale Kommunikationsschaltung ausgebildet, und wird dazu verwendet, die aktuellen gespeicherten Daten des Schrittmachers auf Abruf nach außen zu übermitteln und Modifikationen von außen in den Speicher 66 zu übernehmen.

## Patentansprüche

1. Herzschrittmacher mit einer an die Aktivität eines Patienten angepaßten Taktrate, mit einem Taktgenerator (46) zur Erzeugung von Stimulationsimpulsen für Herzmuskeln am Ende eines Grundraten- oder Escape-Intervalls, einem Aktivitätssensor (56) zum Detektieren von Aktivität des Patienten, einer Auswerteschaltung (57 bis 60) zum Erzeugen eines Steuersignals für die Rate von Stimulationsimpulsen (30, 51) sowie Mitteln zur Beeinflussung des Grundraten- oder Escape-Intervalls zwischen festgelegten Grenzen in Abhängigkeit von der detektierten Aktivität,
**dadurch gekennzeichnet,**
daß ein Schwellwertdiskriminator (57) mit einer Einrichtung (59) zum Programmieren eines Schwellwertes für die Aktivitätssignale vorgesehen ist und diese Rate der Stimulationsimpulse durch das Verhältnis der Zeitdauer des Verweilens des bandbegrenzten Ausgangssignals des Aktivitätssensors (56) oberhalb eines programmierten Schwellwertes zur Zeitdauer des Verweilens unterhalb des Schwellwertes bestimmt wird.

2. Herzschrittmacher nach Anspruch 1, **dadurch gekennzeichnet,** daß die Zeitdauern oberhalb und unterhalb des Schwellwertes vor der Verhältnisbildung jeweils einer gewichteten Mittelwertbildung unterworfen werden.

3. Herzschrittmacher nach Anspruch 2, **dadurch gekennzeichnet,** daß die Gewichtungsfaktoren für die Zeitdauer des Überschreitens des Schwellwertes (59) einerseits und die Zeitdauer des Unterschreitens des Schwellwertes durch das momentane Ausgangssignal des Aktivitätssensors andererseits voneinander verschieden sind.

4. Herzschrittmacher nach Anspruch 3, **dadurch gekennzeichnet,** daß die Gewichtungsfaktoren als programmierbare Größen die Anstiegs- (61) bzw. Abfallrate (62) des ein Zeitmaß für die Stimulationsrate bildenden Steuersignals (Fig. 2) bestimmen.

5. Herzschrittmacher nach einem der Ansprüche 2 bis 4, **dadurch gekennzeichnet,** daß die gewichtete Mittelwertbildung mittels eines dem Bandfilter und dem Schwellwertdiskriminator (57) nachgeschalteteten Integrators (58) erfolgt.

6. Herzschrittmacher nach Anspruch 5, **dadurch gekennzeichnet,** daß der Integrator (58) durch einen Kondensator gebildet wird, der in den durch Über- bzw. Unterschreiten des Schwellwertes festgelegten Zeiten mit vorgegebenen Stromwerten, vorgegebenen Ladungsmengen pro Zeiteinheit oder über vorgegebene Widerstände geladen oder entladen wird, wobei die vorgegebenen Größen gegebenenfalls programmierbar veränderlich sind.

7. Herzschrittmacher nach einem der vorangehenden Ansprüche, **dadurch gekennzeichnet,** daß das Bandfilter für einen Frequenzbereich von 2 bis 8 Hz durchlässig ist.

8. Herzschrittmacher nach Anspruch 4, **dadurch gekennzeichnet,** daß die Anstiegsrate größer gewählt ist als die Abfallrate, wobei diese Werte den entsprechenden physiologischen Werten bei den zu den jeweiligen Steuersignalen gehörigen tatsächlichen körperlichen Belastungen angepaßt sind.

## Claims

1. A heart pacemaker with a clock rate adapted to the activity of a patient, with a clock generator (46) for the generation of stimulation pulses for heart muscles at the end of a basic rate- or escape interval, an activity sensor (56) for the detection of activity of the patient, an analysis circuit (57 to 60) for the generation of a control signal for the rate of stimulation pulses (30, 51) and means for influencing the basic rate- or escape interval between defined limits as a function of the detected activity, characterised in that a threshold value discriminator (57) with a device (59) for programming a threshold value for the activity signals is provided and the rate of the stimulation pulses is defined by the ratio of the dwell time of the band-limited output signal of the activity sensor (56) above a programmed threshold value to the dwell time below the threshold value.

2. A heart pacemaker as claimed in Claim 1, characterised in that the times above and below the threshold value are in each case subjected to a weighted mean value formation prior to the ratio formation.

3. A heart pacemaker as claimed in Claim 2, characterised in that the weighting factors for the time of the overshooting of the threshold value (59) on the one hand and the time of the undershooting of the threshold value by the instantaneous output signal of the activity sensor on the other hand are different to one another.

4. A heart pacemaker as claimed in Claim 3, characterised in that the weighting factors, as programmable variables, define the rise rate (61) and fall rate (62) of the control signal which forms a time scale for the stimulation rate (Fig. 2).

5. A heart pacemaker as claimed in one of Claims 2 to 4, characterised in that the weighted mean value formation is carried out by means of an integrator (58) connected to the output end of the band filter and the threshold value discriminator (57).

6. A heart pacemaker as claimed in Claim 5, characterised in that the integrator (58) is formed by a capacitor which is charged or discharged in the times defined by the overshooting and undershooting of the threshold value with predetermined current values, predetermined charge quantities per time unit or via predetermined resistances, where the predetermined variables optionally can be changed in programmable fashion.

7. A heart pacemaker as claimed in one of the preceding claims, characterised in that the band filter is transmissive for a frequency range of 2 to 8 Hz.

8. A heart pacemaker as claimed in Claim 4, characterised in that the rise rate is selected to be greater than the fall rate, where these values are adapted to the corresponding physiological values for the actual bodily stresses associated with the respective control signals.

## Revendications

1. Stimulateur cardiaque à fréquence de cadencement adaptée à l'activité d'un patient, comportant un générateur de cadencement (46) pour produire des impulsions de stimulation pour les muscles cardiaques à la fin d'un intervalle à fréquence de base - ou d'un intervalle d'échappement, un capteur d'activité (56) pour détecter l'activité du patient, un circuit de traitement (57 à 60) pour produire un signal de commande pour la fréquence des impulsions de stimulation (30, 51), ainsi que des moyens pour influencer l'intervalle à fréquence de base - ou l'intervalle d'échappement - entre des limites déterminées en fonction de l'activité détectée,
caractérisé
par le fait qu'est prévu un discriminateur à valeur de seuil (57) avec un dispositif (59) pour programmer une valeur de seuil pour les signaux d'activité et que cette fréquence des impulsions de stimulation est déterminée par le rapport entre la durée pendant laquelle le signal de sortie, limité en largeur de bande, du capteur d'activité (56) se situe au-dessus d'une valeur de seuil programmée et la durée pendant laquelle ce signal se situe au-dessous de la valeur de seuil.

2. Stimulateur cardiaque selon la revendication 1, caractérisé par le fait que les durées pendant lesquelles le signal se situe au-dessus et au-dessous de la valeur de seuil sont chaque fois soumises, avant la formation du rapport, à la formation d'une valeur moyenne pondérée.

3. Stimulateur cardiaque selon la revendication 2, caractérisé par le fait que les facteurs de pondération pour la durée pendant laquelle les signaux de sortie momentanés du capteur d'activité se situent au-dessus de la valeur de seuil (59) d'une part et la durée pendant laquelle ils se situent au-dessous de la valeur de seuil d'autre part sont différents l'un de l'autre.

4. Stimulateur cardiaque selon la revendication 3, caractérisé par le fait que les facteurs de pondération déterminent, sous forme de valeurs programmables, la fréquence d'attaque (61) ou de décroissance (62) du signal de commande qui forme une mesure du temps pour la fréquence de stimulation (figure 2).

5. Stimulateur cardiaque selon l'une des revendications 2 à 4, caractérisé par le fait que la formation de la valeur moyenne pondérée se fait au moyen d'un intégrateur (58) placé en aval du filtre passe-bande et du discriminateur à valeur de seuil (57).

6. Stimulateur cardiaque selon la revendication 5, caractérisé par le fait que l'intégrateur (58) est formé par un condensateur qui, pendant les périodes déterminées par le fait que le signal dépasse, par valeur supérieure ou inférieure, la valeur de seuil, est chargé ou déchargé avec des valeurs prescrites de l'intensité, des valeurs prescrites de la charge par unité de temps ou par l'intermédiaire de résistances prescrites, les valeurs prescrites pouvant éventuellement être modifiées par programmation.

7. Stimulateur cardiaque selon l'une des revendications précédentes, caractérisé par le fait que le filtre passe-bande laisse passer une bande de fréquence allant de 2 à 8 Hz.

8. Stimulateur cardiaque selon la revendication 4, caractérisé par le fait que la fréquence d'attaque est choisie supérieure à la fréquence de décroissance, ces valeurs étant adaptées aux valeurs physiologiques appropriées correspondant à des efforts physiques effectifs se référant aux signaux de commande respectifs.
